Europäisches Patentamt

European Patent Office

Office européen des brevets

(11) Veröffentlichungsnummer : **0 025 575**
**B1**

# EUROPÄISCHE PATENTSCHRIFT

(45) Veröffentlichungstag der Patentschrift :
09.03.83

(21) Anmeldenummer : 80105344.8

(22) Anmeldetag : 06.09.80

(51) Int. Cl.³ : **B 01 L 11/00**, F 04 B 49/00,
A 61 M 5/14, H 02 P 7/00

(54) **Dosiervorrichtung.**

(30) Priorität : 13.09.79 DE 2937066

(43) Veröffentlichungstag der Anmeldung :
25.03.81 Patentblatt 81/12

(45) Bekanntmachung des Hinweises auf die Patenterteilung : 09.03.83 Patentblatt 83/10

(84) Benannte Vertragsstaaten :
AT BE CH DE FR GB IT LI LU NL SE

(56) Entgegenhaltungen :
DE A 2 529 636
DE B 2 235 208
FR A 2 094 610
FR A 2 185 790
US A 3 173 575
US A 3 701 345
US A 3 771 694
US A 3 978 384
US A 4 111 198

(73) Patentinhaber : BOEHRINGER MANNHEIM GMBH
Sandhofer Strasse 112-132 Postfach 31 01 20
D-6800 Mannheim 31-Waldhof (DE)

(72) Erfinder : Paletta, Benno, Prof. dr. phil.
Buchenhain 10
A-8042 Graz (AT)
Erfinder : Möller, Reinhard, Dr. phil.
Janneckweg 26
A-8042 Graz (AT)
Erfinder : Kasielke, Joachim, Dipl.-Ing.
Kirchenstrasse 13
D-6831 Brühl (DE)

Dosiervorrichtung

Die Erfindung betrifft eine Dosiervorrichtung für Flüssigkeiten, insbesondere in der klinischen Chemie, mit einem mit einem Motor angetriebenen Kolben-Zylinder-System, mit einem optischen Ortsmeßsystem, dessen transparenter, mit undurchsichtigen Markierungen versehener Impulsmaßstab am Kolben befestigt ist, mit einer optisch-digitalen, einen Lichtsender und einen Lichtempfänger zur optischen Abtastung der Impulse einschließenden Ortmeßschaltung, die den Hub des Kolbens in eine entsprechende Anzahl von digitalen Impulsen umformt, und mit einer Steuer-, Rechen- und Vergleicherschaltung, die eine die digitalen Impulse zählende Zählereinrichtung enthält und den Hub des Kolbens in Abhängigkeit von der vorgewählten Einstellung des Dosiervolumens steuert.

Eine derartige Vorrichtung ist aus der DE-B-2 235 208 bekannt. Diese Entgegenhaltung zeigt ein Tischgerät, das ans Lichtnetz angeschlossen werden muß. Das Kolben-Zylinder-System wird durch einen mit Wechselstrom betriebenen Wendemotor mit zwei Wicklungen — je eine für vorwärts und rückwärts — angetrieben. Am Kolben ist ein transparentes Lineal mit vier Markierungen, entsprechend den Mengen von 25, 50, 100 oder 200 Mikroliter, angebracht. Diese Markierungen werden optisch abgetastet. Zu diesem Zweck läuft das transparente Lineal zwischen einem Lichtsender und einem als Differential-Photodiode ausgebildeten Lichtempfänger. Die Information, welche der vier möglichen Mengen dosiert werden soll, wird mit Hilfe einer Lochkarte in das Tischgerät einprogrammiert. Sobald die vorprogrammierte Markierung von den Photozellen erkannt worden ist, wird der Motorstrom abgeschaltet, und es bleibt der Reibung des Getriebes und des Kolbens überlassen, das Schwungmoment des Motors abzubremsen.

Eine weitere Dosiervorrichtung ist aus der FR-A 2 185 790 bekannt. Bei dieser Vorrichtung wird die Bewegung des Kolbens auf eine Scheibe übertragen, die mit einem regelmäßigen Strichgitter versehen ist. Diese Scheibe wird durch eine ebenfalls mit einem regelmäßigen Strichgitter versehene Abtastplatte hindurch beleuchtet. Ein solches optisches Ortsmeßsystem ist im Prinzip zwar wesentlich genauer als das in der DE-B 2 235 208 verwendete, jedoch wird diese Genauigkeit durch den zwischen der Scheibe und dem Kolben auftretenden, wenn auch geringen Schlupf verringert.

In der FR-A 2 185 790 wird ausführlich beschrieben, daß Genauigkeitsprobleme außerdem durch das Nachlaufen von Dosierflüssigkeit bei bereits stromlos gemachtem Antriebsmotor entstehen. Dieses Nachlaufen von Dosierflüssigkeit wird dadurch berücksichtigt, daß der Zähler, in den die von der sich drehenden Codierscheibe kommenden Impulse eingezählt werden, vor Beginn des Dosiervorganges auf eine bestimmte Zahl von Impulsen voreingestellt wird. Da diese voreinzustellende Zahl sehr schwierig auszurechnen ist, ist eine solche Lösung im normalen Alltagslaborbetrieb indiskutabel. Außerdem wird dadurch der Aufwand an Schaltungselektronik vergrößert, da zusätzlich ein Zähler und ein Zählstandsvergleicher benötigt werden.

Aus der US-A 3 701 345 ist eine Injektionseinrichtung für die Angiographie, d. h. die Darstellung von Blutgefäßen im Röntgenbild bekannt, bei der zur Steuerung des abgegebenen Volumens ein mit dem Antriebsmotor mechanisch verbundenes Potentiometer verwendet wird. Dieses Potentiometer liefert den Ist-Wert der Motoreinstellung, der mit Hilfe einer Regeleinrichtung mit dem als Spannung vorgegebenen Soll-Wert verglichen wird, um die Einstellung des mit dem Motor verbundenen Kolbens zu steuern. Wie bei allen Einrichtungen, bei denen der Ist-Signalgeber mit dem Antriebsmotor statt mit dem Kolben zusammenwirkt, beeinflussen auch hier Ungenauigkeiten und das unvermeidliche Spiel zwischen den Elementen der Kraftübertragung vom Motor auf den Kolben die Genauigkeit der Volumendosierung, so daß schon aus diesem Grund diese vorbekannte Einrichtung für die Zwecke der vorliegenden Erfindungen ungeeignet ist. Bei dieser Vorrichtung wird ein Gleichstrommotor verwendet, der sich durch ein hohes Drehmoment bei kompakter und leichter Bauweise auszeichnet. Ein derartiger Gleichstrommotor läßt sich vorteilhafterweise im Rahmen einer Vierquadrantensteuerung einsetzen, wie sie für den Fall einer Magnetbandsteuerung beispielsweise aus der US-A 3 978 384 zu entnehmen ist.

In allen Laboratorien, in welchen Serienanalysen durchgeführt werden, müssen die verschiedenen Arbeitsabläufe, insbesondere die zahlreichen Dosiervorgänge, ständig weiter rationalisiert werden. Die Verwendung von Analysenvollautomaten erfordert hohe Investitionskosten, eine jederzeit ökonomische Auslastung der Anlage und speziell ausgebildetes Personal. Treten in derart komplexen Anlagen Fehler auf, so entstehen kostspielige Wartezeiten. Es wurden deshalb elektronische Dosiergeräte entwickelt, bei welchen entweder über digital gesteuerte Schrittmotoren oder, wie oben beschrieben, über mit optischen Meßsystemen gesteuerte Motoren die Kolben der Dosierspritzen bewegt werden. Größe und Gewicht der im letzterem Fall bisher verwendeten Wechselstrommotoren sowie der mechanischen Kraftübertragung führen jedoch zu einer unbequemen Handhabung der üblicherweise als Standgeräte ausgebildeten Apparate. Außerdem sind diese Geräte auf einen stetigen Netzanschluß angewiesen. Größe und Gewicht dieser Geräte wirken sich auch auf den Preis der Gesamtkonstruktion aus, insbesondere wenn sehr hohe Genauigkeitsanforderungen gestellt werden.

Der vorliegenden Erfindung liegt deshalb die Aufgabe zugrunde, eine Dosiervorrichtung der eingangs genannten Art anzugeben, welche ohne schwere und teuere Präzisionsmechanik auskommt, eine extrem hohe Präzision der zu dosierenden Volumina ermöglicht, alle Dosiervorgänge wie Dispensieren, Dilutieren, Pipettieren, Titrieren etc. durchzuführen gestattet und einen so geringen Stromverbrauch hat, daß sie gegebenenfalls durch eingebaute Akkumulatoren oder Batterien betrieben werden kann. Sie soll für Handgeräte ebenso wie für stationär einsetzbare Geräte verwendbar sein.

Diese Aufgabe wird dadurch gelöst, daß benachbart zum ersten Impulsmaßstab ein weiterer Impulsmaßstab als Abtastplatte in einer relativ zum Zylinder festen Position angeordnet ist, daß die Markierungen auf den Impulsmaßstäben ein regelmäßiges Strichgitter bilden, daß der Motor ein Gleichstrommotor ist, daß der Motor in Abhängigkeit vom Stand der Zähleinrichtung in seiner Drehzahl gesteuert ist, derart, daß bei Annäherung an das vorgewählte Dosiervolumen eine niedrigste Geschwindigkeitsstufe eingestellt ist, und daß ein Schaltungsteil zum schlagartigen Stillsetzen des Motors unter Anlegen einer Gegenspannung bei Erreichen des dem vorgewählten Dosiervolumen entsprechenden Zählerstandes vorgesehen ist.

Damit ergeben sich die Vorteile, daß die Volumenmessung mit außergewöhnlicher Genauigkeit durchgeführt werden kann, daß das Getriebe, welches die Drehbewegung des Motors in die Kolbenbewegung umsetzt, sehr einfach und preiswert ausgeführt werden kann, und daß der Kolbenhub unmittelbar optisch mit hoher Präzision gemessen werden kann. Als weiterer wesentlicher Vorteil ist anzusehen, daß durch die Verwendung eines Gleichstrommotors, vorzugsweise eines kleinen Glockenankermotors, die Kolbenhubpipette und ihr mechanischer Antrieb so klein und leicht ausgeführt werden können, daß sie bequem in der Hand gehalten werden kann und trotzdem die vorteilhaften Eigenschaften herkömmlicher, wesentlich größerer Geräte wie hohe Positioniergenauigkeit und großes Haltemoment besitzt oder übertrifft. Dies ist insbesondere darauf zurückzuführen, daß die erfindungsgemäße elektronische Steuerschaltung ein schlagartiges Stillsetzen der Motordrehbewegung ermöglicht, so daß die durch die verwendete optische Messung gewährleistete hohe Meßgenauigkeit auch in eine entsprechend hohe Dosiergenauigkeit umgesetzt werden kann.

Das zur Messung des Kolbenhubs verwendete optische Meßsystem wird als digital-inkrementales Meßsystem bezeichnet. Es ist prinzipiell bekannt, beispielsweise aus der DE-Zeitschrift « BBC-Nachrichten », September 1967, Seiten 464 bis 471, insbesondere Seite 468. Obwohl sich derartige Systeme in vielen Bereichen der Technik bereits bewährt haben und eine Auflösung bis herunter zu einem Mikrometer erlauben, ist eine Verwendung derartiger Geräte in Form eines geraden Impulsmaßstabs bei Dosiervorrichtungen vorliegender Art nicht bekannt geworden.

Vorzugsweise ist der Lichtsender im optischen Ortsmeßsystem eine Lumineszensdiode, der Lichtempfänger ein Phototransistor, ist dem Lichtempfänger ein Spannungskomparator nachgeschaltet und zwischen Lichtempfänger und Spannungskomparator ein Differenzierglied eingeschaltet. Diese Komponenten bilden zusammen einen Analog-Digital-Wandler, der das von dem Photoempfänger aufgenommene optische Analogsignal in ein Digitalsignal umwandelt, welches zur Weiterverarbeitung in der digitalen Steuer-, Rechen- und Vergleicherschaltung geeignet ist.

Gemäß einer vorteilhaften Ausgestaltung der Erfindung enthält die Steuer, Rechen- und Vergleicherschaltung eine Drehzahlregelung, die den Motor auf die vorgewählte Geschwindigkeitsstufe beschleunigt und ihn eine bestimmte Zahl von optischen Impulsschritten vor Erreichen des vorgewählten Volumens auf die niedrigste Geschwindigkeitsstufe verzögert. Auf diese Weise lassen sich eine hohe Dosiergeschwindigkeit und gleichzeitig eine hohe Dosiergenauigkeit erreichen. Ohne ein Verzögern des Motors auf die niedrigste Geschwindigkeitsstufe vor Erreichen des eingestellten Volumens könnte auch mit der noch zu beschreibenden erfindungsgemäßen Bremsschaltung nicht die geforderte hohe Dosiergenauigkeit erreicht werden.

Vorzugsweise enthält die Drehzahlregelung eine geregelte Spannungsquelle, deren Ausgangsspannung die Motorbetriebsspannung ist und deren Sollwert in mehreren Stufen einstellbar ist ; außerdem ist die Drehzahlregelung bei einem kleinen zu verarbeitenden Volumen immer auf die niedrigste Geschwindigkeitsstufe eingestellt. Während der Stillsetzphase bekommt der Antriebsmotor kurzzeitig eine Gegenspannung zugeführt, wobei jedoch die maximal zulässige Motorbetriebsspannung nicht überschritten wird, um die Motorlebensdauer nicht herabzusetzen.

Gemäß einer bevorzugten Weiterbildung der Erfindung wird während des Stillsetzens des Motors unter Anlegen einer Gegenspannung diese Gegenspannung periodisch abgeschaltet. Während der Motor frei läuft, wird die Leer-laufspannung gemessen, wobei das Verschwinden der Leerlaufspannung den Stillstand des Motors anzeigt. Diese Messungen erfolgen fortlaufend und in kurzen Zeitabständen, so daß ein Überbremsen des Motors und damit ein Rückwärtslaufen desselben vermieden wird oder mindestens so geringfügig ist, daß die Genauigkeit der Pipettierung nicht darunter leidet.

Dabei sei angemerkt, daß das Getriebe bei der erfindungsgemäßen Pipettensteuerung mit relativ geringer Präzision gefertigt sein und ein gewisses Spiel aufweisen darf.

Zur Messung der Motorleerlaufspannung ist ein Nullspannungskomparator vorgesehen, der bei Verschwinden der Leerlaufspannung ein Ausgangssignal abgibt. Der Meßeingang des Nullspannungskomparators ist vorzugsweise über einen Spannungsteiler mit den Motoranschlüssen verbunden. Der Nullspannungskomparator ist vorzugsweise ein entsprechend geschalteter Operationsverstärker. Die Anschaltung des Komparators mit Hilfe eines Spannungsteilers hat den Vorteil, daß für beide Motordreheinrichtungen nur ein einziger Komparator verwendet werden muß, da die Mittelspannung des Spannungsteilers immer die gleiche Polarität gegenüber der Bezugselektrode der Schaltung besitzt. Die Tatsache, daß durch die Verwendung eines Spannungsteilers die dem Meßeingang des Komparators zugeführte Spannung halbiert ist, kann leicht durch eine entsprechende Erhöhung der Verstärkung des Komparators ausgeglichen werden.

Vorzugsweise ist der Motor in einer Brückenschaltung von vier Transistoren angeordnet. Durch das Einschalten von jeweils zwei diagonal zueinander liegenden Transistoren kann der Motor von Vorwärts- auf Rückwärtslauf umgeschaltet werden, ohne daß die an die Brückenschaltung gelegte Motorbetriebsspannung umgepolt werden müßte.

Vorteilhafterweise ist der Transistor-Brückenschaltung ein Betriebsartenumschalter zum Umschalten des Motors auf Vorwärts- oder Rückwärtslauf vorgeschaltet. Dem Betriebsartenumschalter ist eine Motorsteuerlogik vorgeschaltet, die in der Motorbetriebsphase jeweils zwei diagonal zueinander liegende Transistoren einschaltet, während der Motorstillsetzphase einen der beiden Transistoren periodisch kurzzeitig abschaltet und bei Motorstillstand die Transistoren so einschaltet, daß der Motor kurzgeschlossen ist. Die Motorsteuerlogik bildet aus den von der Steuerschaltung vorgegebenen Signalen wie « Motor antreiben », « Motor stillsetzen », « Motor Drehzahl messen » die Ansteuerimpulse für die Transistoren der Brückenschaltung. Der Betriebsartenumschalter seinerseits verteilt jenachdem, ob der Motor vorwärts oder rückwärts drehen soll, diese Ansteuerimpulse auf die Transistoren, die die gewünschte Motorfunktion ermöglichen.

Gemäß einer vorteilhaften Weiterbildung ist in der Stromzuführung zur Transistorbrückenschaltung ein Widerstand angeordnet, und die an diesem Widerstand abfallende Spannung dient als Istwert für die Drehzahlregelung. Die an diesem Widerstand abfallende Spannung, die dem Strom durch den Motor direkt proportional ist, kann außerdem dazu benutzt werden, den Motorstrom auf den zulässigen Maximalwert zu begrenzen.

Gemäß einer vorteilhaften Weiterbildung der Erfindung ist zum periodischen, kurzzeitigen Abschalten eines der beiden, den Bremsstrom führenden Transistoren ein Taktgenerator vorgesehen, der stark asymmetrische Impulse abgibt, wobei die langen Impulse die Bremsphase, die kurzen Impulse die Meßphase definieren. Während der Dauer der kurzen Impulse ist während der Motorstillsetzphase der eine der beiden Bremsstrom führenden Transistoren abgeschaltet ; während der Dauer der langen Impulse bleibt er eingeschaltet. Das bedeutet, daß während der längeren Zeitspanne der Motor aktiv gebremst wird und während einer kurzen Zeitspanne von der Motorbetriebsspannung abgetrennt wird und leerläuft.

Vorzugsweise ist eine Meß- und Stopp-Logik vorgesehen, die während der Dauer der kurzen Impulse des Taktgenerators den Meßzeitpunkt festlegt und während der Motorstillsetzphase so lange Impulse an die Motorsteuerlogik weiterleitet, bis das Nullspannungssignal des Nullspannungskomparators erscheint. Mit Hilfe dieses Schaltungsteiles wird erreicht, daß in der Stillsetzphase nach dem Abschalten des einen der beiden Bremsstrom führenden Transistoren und der während dieser Zeitspanne erfolgenden Messung der Leerlaufspannung des Motors die Abfrage des Meßergebnisses zunächst um eine gewisse kurze Zeitspanne verzögert wird, da sonst die Gefahr besteht, daß nicht die Leerlaufspannung sondern die durch das Abschalten des Bremsstromes im Motor entstehende induktive Spannungsspitze ausgewertet würde, was zu einem völlig falschen Ergebnis führt.

Vorteilhafterweise umfaßt die Zähleinrichtung einen extern voreinstellbaren Zähler, eine extern bedienbare Hand-Start-einrichtung für den Zähler, einen Zählereingang für die Impulse von dem Ortsmeßsystem und eine Einrichtung zum Erkennen kleiner Volumina, deren Ausgang mit der Drehzahlregelschaltung verbunden ist und diese auf die kleinste Geschwindigkeitsstufe einstellt. Die Voreinstellung des zu verarbeitenden Volumens erfolgt beispielsweise mit Hilfe von mehreren Dekadenwahlschaltern. Sobald die Starttaste betätigt wird, um einen der Dosiervorgänge durchzuführen, sorgt die Starteinrichtung dafür, daß zunächst die an den Dekadenwahlschaltern eingestellten Zahlen in den Zähler übernommen werden, daß dann der Motor über die Motorsteuerlogik, den Betriebsartenumschalter sowie die Drehzahlregelschaltung gestartet wird, daß die Drehzahlregelschaltung bei kleinen Volumina — sei es, daß diese von vornherein eingestellt waren, sei es, daß sich der Kolben inzwischen seinem Zielwert genähert hat — auf die niedrigste Geschwindigkeitsstufe eingestellt wird, daß die von der optischen Meßeinrichtung für den Kolbenvorschub kommenden Impulse von dem eingestellten Zählerstand abgezogen werden und daß bei Zählerstand Null die Meß- und Stopplogik aktiviert wird.

Gemäß einer vorteilhaften Weiterbildung ist eine vollautomatisch wirkende Kolbenstartpunkt-Sucheinrichtung vorgesehen, die die Zahl der pro Zeiteinheit erzeugten optischen Impulsschritte überwacht und den Motorstrom abschaltet, wenn pro Zeiteinheit keine Impulse erzeugt werden. Mit einer derartigen Einrichtung ist es möglich, den Kolben einer in das Handgerät eingesetzten Pipette definiert auf den Nullpunkt zu setzen, unabhängig von der aktuellen Länge der Pipette oder deren Kolben. Mit Hilfe dieser Einrichtung ist es weiterhin möglich, den Kolben mit Hilfe des Motors vollautomatisch bis zum Anschlag an den Zylinder vorzufahren. Außerdem ermöglicht es diese Einrichtung, bei einer Störung des normalen Kolbenvorschubs den Motor abzuschalten, bevor eine Überlastung von Motor oder Getriebe eintritt. Beim Ausbleiben der optischen Impulse erzeugt die Kolbenstartpunkt-Sucheinrichtung einen Nullsetzimpuls, der den Zähler auf Null zurücksetzt und damit den Motor-Stillsetzvorgang einleitet.

Gemäß einer bevorzugten Ausführungsform der Erfindung wird die Zahl der Ausgangsimpulse des Ortsmeßsystems pro Zeiteinheit als Maß der Kolbengeschwindigkeit zur Regelung der Drehzahl des Motors verwendet. Dies hat den Vorteil, daß die Geschwindigkeit des Kolbens durch das Ortsmeßsystem besonders genau gemessen wird und erleichtert eine digitale Signalverarbeitung.

Gemäß einer Weiterbildung ist ein optischer Impulsintegrator vorgesehen, der die Ausgangsimpulse des optischen Ortsmeßsystems integriert und als Istwert der Kolbengeschwindigkeit an die geregelte Spannungsquelle für die Motorbetriebsspannung weiterleitet. Bei einer Variante wird die Kolbengeschwindigkeit direkt geregelt und nicht auf dem Umweg über den Motorstrom.

Gemäß einer bevorzugten Ausgestaltung ist die geregelte Spannungsquelle für die Motorbetriebsspannung als getaktete Spannungsquelle mit einstellbarem Impuls-Pausen-Verhältnis ausgebildet. Eine getaktete Spannungsquelle hat einen sehr hohen Wirkungsgrad und einen kleinen Ausgangswiderstand. Der hohe Wirkungsgrad ist von besonderem Vorteil, wenn das Dosiergerät aus Akkumulatoren oder Batterien betrieben wird. Der kleine Ausgangswiderstand unterstützt insbesondere beim Stillsetzvorgang das schlagartige Abbremsen des Motors und trägt damit zur hohen Dosiergenauigkeit bei. Außerdem ist eine derartige Spannungsquelle als integrierter Schaltkreis handelsüblich.

Besonders vorteilhaft wird es, wenn die Transistoren der Brückenschaltung gleichzeitig Teil der getakteten Spannungsquelle sind. Auf diese Weise wird der Aufwand für die elektronische Schaltung minimal.

Anhand der Zeichnung soll die Erfindung in Form von Ausführungsbeispielen näher erläutert werden. Es zeigen :

Figur 1 ein Blockschaltbild der elektronischen Steuer-, Rechen- und Vergleicherschaltung einer Dosiervorrichtung,

Figur 2 ein Blockschaltbild eines optischen Ortsmeßsystems mit Impulsformung, Kolbenstartpunktsucheinrichtung, Zähleinrichtung und Impulsintergrierschaltung,

Figur 3 eine mögliche Realisierung für den Schaltungsteil der Fig. 2,

Figur 4 ein Blockschaltbild des Teils der Steuer-, Rechen- und Vergleicherschaltung, der für den Betrieb des Motors in Vorwärts- und Rückwärtslauf sowie für das Abbremsen und Stillsetzen zuständig ist,

Figur 5 eine erste mögliche Realisierung für den Schaltungsteil der Fig. 4,

Figur 6 ein Blockschaltbild der Drehzahlregelung,

Figur 7 eine erste mögliche Realisierung der Drehzahlregelung,

Figur 8 eine mögliche Realisierung für eine automatische Kolbenstartpunktsucheinrichtung,

Figur 9 ein Diagramm, aus dem der Verlauf der Motordrehzahl bei verschiedenen zu verarbeitenden Dosiervolumina und verschiedenen Dosiergeschwindigkeiten ersichtlich ist.

Figur 10 eine Realisierung für einen optischen Impulsintegrator,

Figur 11 eine zweite mögliche Realisierung für den Schaltungsteil der Fig. 4,

Figur 12 eine zweite mögliche Realisierung der Drehzahlregelung.

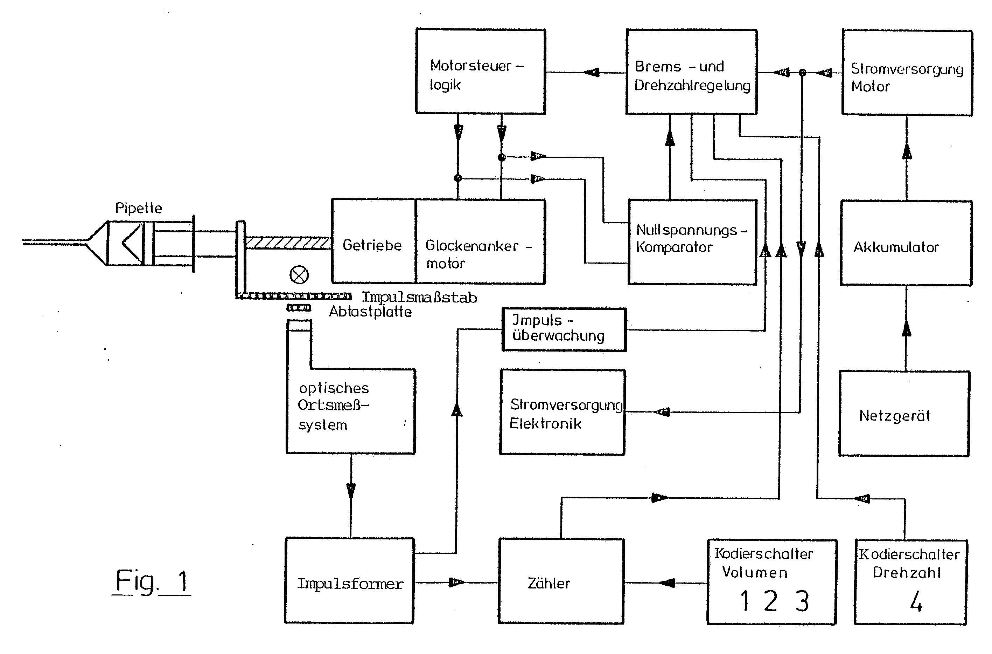

In dem in Fig. 1 dargestellten Blockschaltbild erkennt man links eine Pipette, bestehend aus einem Zylinder, einem darin beweglichen Kolben sowie einer vorzugsweise auswechselbaren Einwegspritze. Der Kolben wird über ein Getriebe von einem Klein-Gleichstrommotor angetrieben. Am Kolben ist ein gerader Impulsmaßstab befestigt, welcher von einem Lichtsender durchstrahlt wird. Auf der dem Lichtsender abgewandten Seite des Impulsmaßstabs befindet sich ein Lichtempfänger, der zu einem optischen Ortsmeßsystem gehört. Zwischen Lichtempfänger und Impulsmaßstab befindet sich zusätzlich ein weiterer Impulsmaßstab als feststehende Abtastplatte. Abtastplatte und Impulsmaßstab sind transparent und mit einem regelmäßigen, undurchsichtigen Strichgitter versehen. Diese Striche verlaufen im wesentlichen senkrecht zur Bewegungsrichtung des Impulsmaßstabs, wobei gewisse Winkelabweichungen durchaus zulässig sind. Von der Genauigkeit des Strichrasters ist im wesentlichen die Genauigkeit der Kolbenbewegung und damit der Dosierung abhängig.

Statt der beschriebenen transmissiven Abtastung, bei der das Meßlicht die beiden Impulsmaßstäbe durchdringt, kann auch eine reflexive Abtastung vorgesehen sein, bei der Lichtsender und Lichtempfänger auf der gleichen Seite der beiden Impulsmaßstäbe angeordnet sind. In diesem Fall ist bevorzugt, aber nicht notwendigerweise der von Lichtsender und Lichtempfänger abgewandte Impulsstab nicht transparent.

Die vom optischen Ortsmeßsystem erzeugten elektrischen Impulse werden in einem zugehörigen Impulsformer in für die digitale Verarbeitung geeignete Form gebracht und einem Zähler zugeführt. Dieser Zähler ist über Kodierschalter voreinstellbar ; er zählt von der voreingestellten Zahl so lange rückwärts, bis die Zahl O erreicht ist.

Der aktuelle Stand des Zählers steuert eine Brems- und Drehzahlregelung. Die Drehzahlregelung kann über einen weiteren Kodierschalter auf verschiedene Geschwindigkeitsstufen eingestellt werden. Über die Drehzahlregelung wird eine Motorsteuerlogik beeinflußt, die den Motor mit der Betriebsspannung versorgt. Die Spannung an den Anschlußklemmen des Motors wird über einen Nullspannungskomparator gemessen, der während der Stillsetzphase des Motors die Bremsspannungsregelung derart beeinflußt, daß der Motor mit Gegenspannung aktiv gebremst wird, daß die Bremsspannung sofort abgeschaltet wird, wenn der Motor stillsteht und daß anschließend die Motoranschlußklemmen kurzgeschlossen werden.

Zur Stromversorgung der Elektronik sowie des Motors dient ein Netzgerät, welches in den Betriebspausen einen Akkumulator auflädt, der seinerseits während des Betriebs die Stromversorgung für den Motor sowie die Stromversorgung für die Elektronik speist.

Zwischen Impulsformer und Brems- und Drehzahlregelung ist ferner eine Impulsüberwachung eingeschaltet. Diese sorgt dafür, daß die Zahl der optischen Impulse pro Zeiteinheit, die ein Maß für die Kolbengeschwindigkeit ist, an die Drehzahlregelung gemeldet wird, wo sie mit dem mit einem Kodierschalter für die Drehzahl eingestellten Wert verglichen wird.

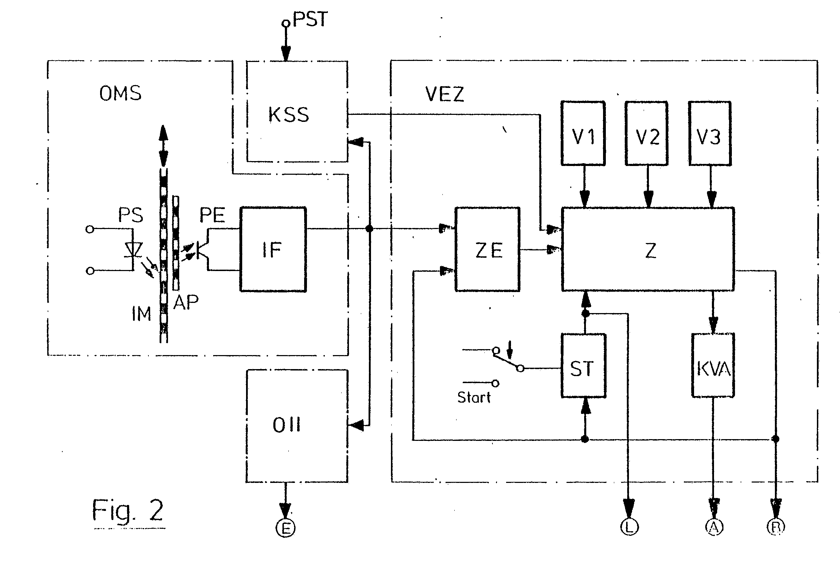

In Fig. 2 erkennt man als Blockschaltbild das optische Ortsmeßsystem OMS mit Lichtsender PS, Impulsmaßstab IM, Abtastplatte AP, Lichtempfänger PE und Impulsformer IF. Man erkennt weiterhin die voreinstellbare Zähleinrichtung VEZ, enthaltend einen Zähler Z, drei Kodierschalter V1, V2, V3, eine Zählereingangsschaltung ZE, eine Startschaltung ST sowie eine Erkennungsschalteinrichtung KVA für kleine Volumina. Die voreinstellbare Zähleinrichtung VEZ gibt drei Ausgangssignale ab, und zwar ist der Ausgang der Erkennungsschalteinrichtung KVA für kleine Volumina an eine Klemme A, der Null-Ausgang des Zählers Z auf eine Klemme B und der Ausgang der Startschaltung ST auf eine Klemme L gelegt. Am Eingang der Zähleingangsschaltung ZE liegen die Impulse des Ortsmeßsystems OMS sowie der Nullausgang des Zählers Z an. Die Zählereingangsschaltung ZE läßt die von dem Ortsmeßsystem OMS kommenden Impulse so lange durch, bis der Zähler Z auf O zurückgezählt ist und der Kolben den voreingestellten Weg zurückgelegt hat.

Ferner erkennt man eine automatische Kolbenstartpunktsucheinrichtung KSS, an deren Eingang die Impulse der Ortsmeßsystems OMS anliegen und deren Ausgang mit dem Nullsetzeingang des Zählers Z verbunden ist. Der Startbefehl zum Auslösen der Kolbenstartpunktsuche wird über einen Programmstarteingang PST gegeben.

Weiterhin erkennt man eine optische Impulsintegrierschaltung OII, an deren Eingang die Ausgangsimpulse der automatischen Kolbenstartpunktsucheinrichtung KSS anliegen. Die optische Impulsintegrierschaltung OII gibt ein Ausgangssignal auf eine Klemme E, welches der Zahl der pro Zeiteinheit eintreffenden optischen Impulse und damit der Geschwindigkeit, mit der der mit dem Kolben verbundene Impulsmaßstab IM bewegt wird, direkt proportional ist.

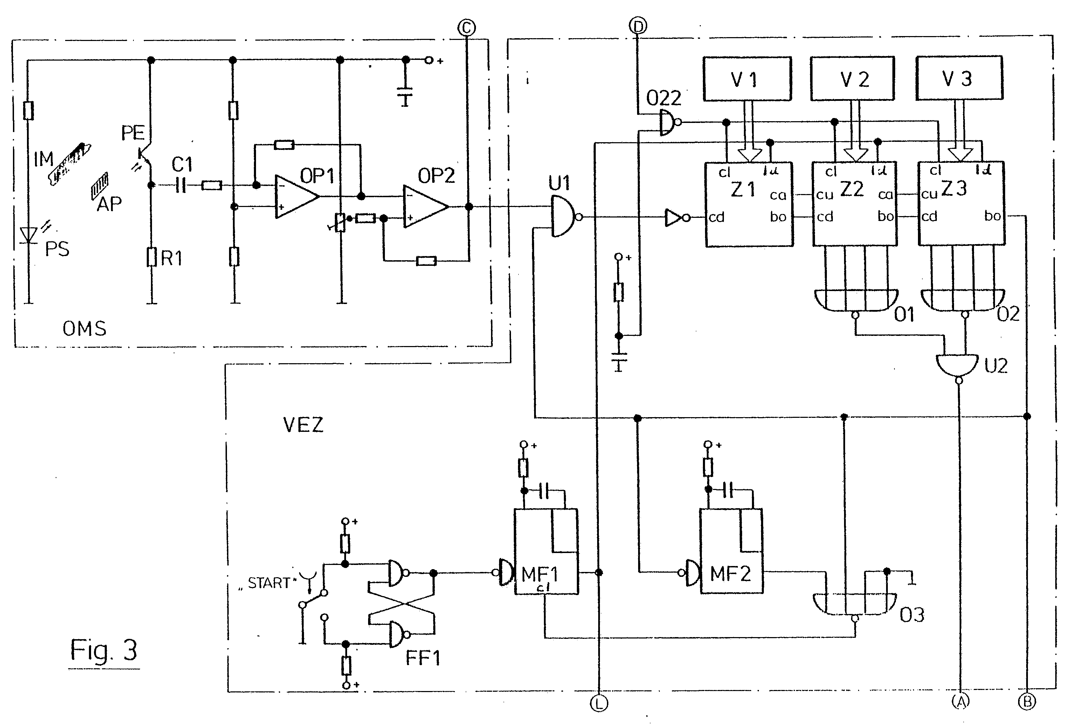

Fig. 3 zeigt eine zu dem in Fig. 2 dargestellten Blockschaltbild des Ortsmeßsystems OMS und des voreinstellbaren Zählers VEZ gehörende reale Schaltung. Im Inneren des durch eine strichpunktierte Linie begrenzten Ortsmeßsystems OMS erkennt man als Lichtsender PS eine Luminiszensdiode, die den Impulsmaßstab IM sowie die Abtastplatte AP durchleuchtet. Das Licht wird von einem als Lichtempfänger PE dienenden Phototransistor aufgenommen und in eine analoge Spannung umgewandelt. Die Ausgangsspannung des Phototransistors wird über ein Differenzierglied R1, C1 auf einen als Verstärker geschalteten ersten Operationsverstärker OP1 und von diesem auf einen als Spannungskomparator geschalteten Operationsverstärker OP2 gegeben. Die am Ausgang des Spannungskomparators OP2 anstehenden Impulse werden auf eine Klemme C, die mit dem Eingang der automatischen Kolbenstartpunktsucheinrichtung KSS verbunden ist und auf die voreinstellbare Zähleinrichtung VEZ gegeben. An dere Eingang liegt ein Nicht-UND-Gatter U1, dessen zweiter Eingang mit dem Ausgang des Hunderter-Dekadenzählers Z3 verbunden ist. Die das Gatter U1 durchlaufenden Impulse werden auf den Einer-Dekadenzähler Z1 gegeben, von diesem auf den Zehner-Dekadenzähler Z2 und von diesem auf den Hunderter-Dekadenzähler Z3. Alle drei Zähler Z1, Z2 und Z3 sind über als Dekadenwahlschalter ausgebildete Kodierschalter V1, V2, V3 voreinstellbar. Die Dual-Ausgänge des Zehner-Dekadenzählers Z2 und des Hunderter-Dekadenzählers Z3 sind auf je ein Nicht-ODER-Gatter 01, 02 geschaltet. Beide Nicht-ODER-Gatter 01, 02 sind über ein Nicht-UND-Gatter U2 verknüpft und an die Ausgangsklemme A geführt.

Die Hand-Starteinrichtung besteht aus einer Starttaste, die über ein nachgeschaltetes Flipflop FF1 entprellt wird. Der Startimpuls des Flipflop FF1 wird über ein Monoflop MF1 mit Sperreingang in einen Startimpuls definierter Zeitdauer umgewandelt Der Sperreingang verhindert, daß unbeabsichtigt mehrere Dosiervorgänge hintereinander gestartet werden, wenn versehentlich die Starttaste mehrfach gedrückt wird oder die Starttaste stark prellt. Das Startsignal wird auf die drei Dekadenzähler Z1, Z2, Z3 geleitet und veranlaßt die Übernahme der an den Schaltern V1, V2, V3 eingestellten Zahlen in die Zähler Z1, Z2, Z3. Gleichzeitig wird das Startsignal an die Klemme L gelegt. Das Sperrsignal für das Monoflop MF1 wird über ein Nicht-ODER-Gatter 03 und ein diesem parallelgeschaltetes Monoflop MF2 erzeugt. Man erkennt in der Schaltung weiterhin noch ein Nicht-ODER-Gatter 022, dessen erstem Eingang ein RC-Glied vorgeschaltet ist, wodurch die Zähler Z1, Z2, Z3 beim Einschalten der Betriebsspannung definiert auf O gesetzt werden.

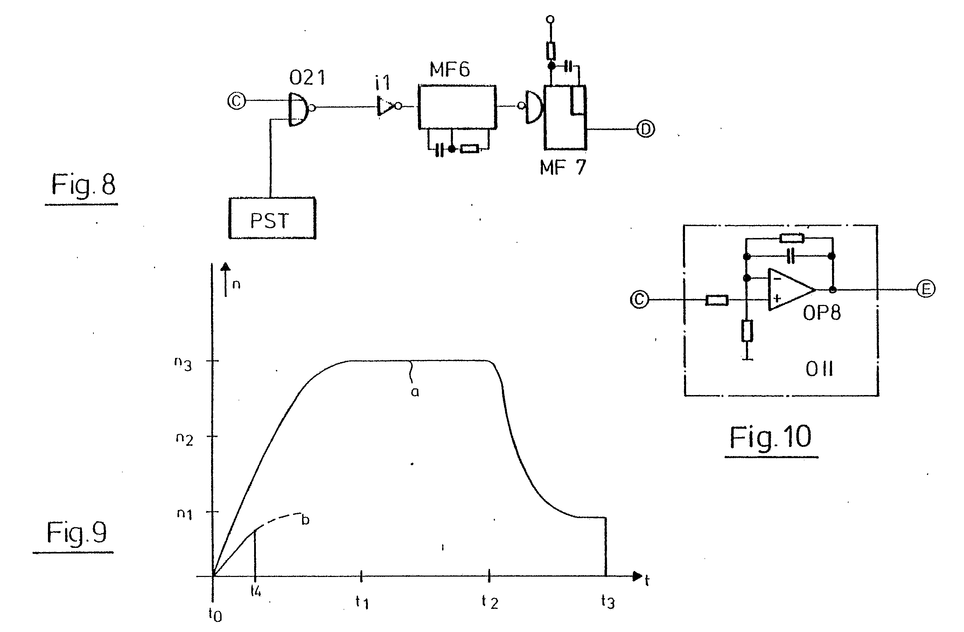

Über den zweiten Eingang des Gatters 022 wird das von der Klemme D kommende Ausgangssignal der automatischen Kolbenstartpunktsucheinrichtung KSS auf die Löscheingänge cl der Zähler Z1, Z2, Z3 gegeben. Aufbau und Wirkungsweise der automatischen Kolbenstartpunktsucheinrichtung KSS wird anhand der Fig. 8 erläutert werden.

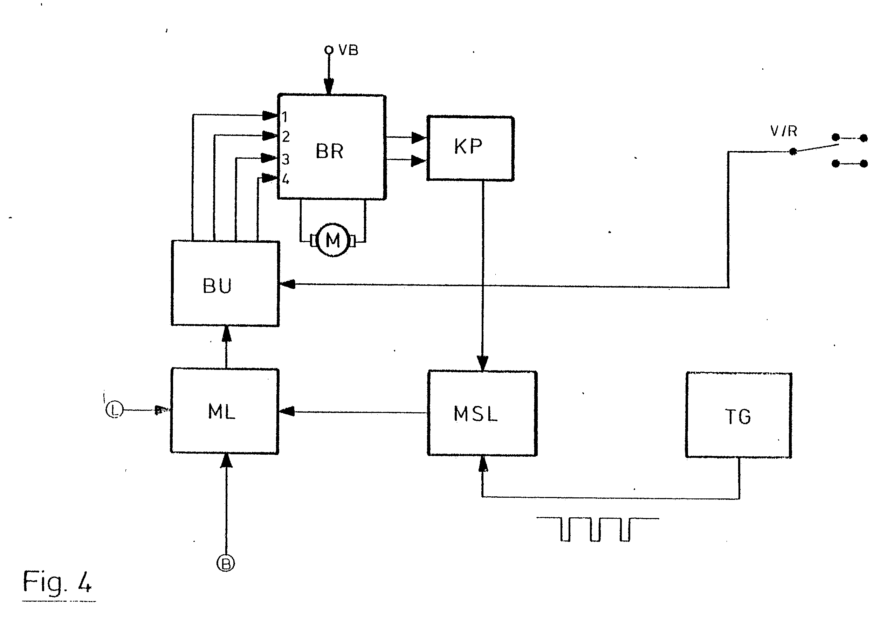

Fig. 4 zeigt den Teil der Steuer-, Rechen- und Vergleicherschaltung, der für die Betriebsarten

« Antreiben », « Bremsen » und « Stillsetzen » des Motors zuständig ist. Der an der Klemme L anstehende Startimpuls wird auf eine Motorsteuerlogik ML gegeben, deren Ausgangssignal über einen Betriebsartenumschalter BU in vier Ansteuersignale für eine Brückenschaltung BR umgewandelt wird. Mit der Brückenschaltung BR ist der Gleichstrommotor M verbunden. Außerdem wird der Brückenschaltung BR eine Motorbetriebsspannung VB zugeführt.

Der Motorsteuerlogik ML wird auch das an der Klemme B anstehende Bremssignal zugeführt. Der Betriebsartenumschalter BU wird von dem Vorwärts-Rückwärts-Signal V/R beeinflußt. Der V/R-Schalter wird entweder vom Bedienungspersonal oder automatisch in Abhängigkeit vom eingestellten Dosierprogramm eingestellt.

Die Motorleerlaufspannung wird von einem Nullspannungskomparatoren KP gemessen. Der Ausgang des Nullspannungskomparators KP wird an eine Meß- und Stopp-Logik MSL geschaltet. An dieser Meß- und Stopp-Logik MSL stehen die Ausgangsimpulse eines Taktgenerators TG an. Während der Stillsetzphase des Motors, d. h. wenn das Bremssignal an der Klemme B anliegt, werden die Ausgangsimpulse des Takgenerators TG so lange über die Meß- und Stopp-Logik MSL an die Motorsteuerlogik ML geliefert, bis von dem Nullspannungskomparator KP das Signal kommt, welches anzeigt, daß der Motor M stillsteht. In diesem Moment werden die Ausgangsimpulse des Taktgenerators TG gesperrt und die Motorsteuerlogik ML so beeinflußt, daß der Motor M kurzgeschlossen wird. Das Kurschließen des stillstehenden Motors M verleiht diesem einen erhöhten Widerstand gegen mechanische Bewegung, ausgelöst beispielsweise durch eine zwangsweise Verschiebung des Kolbens in der Dosierpipette.

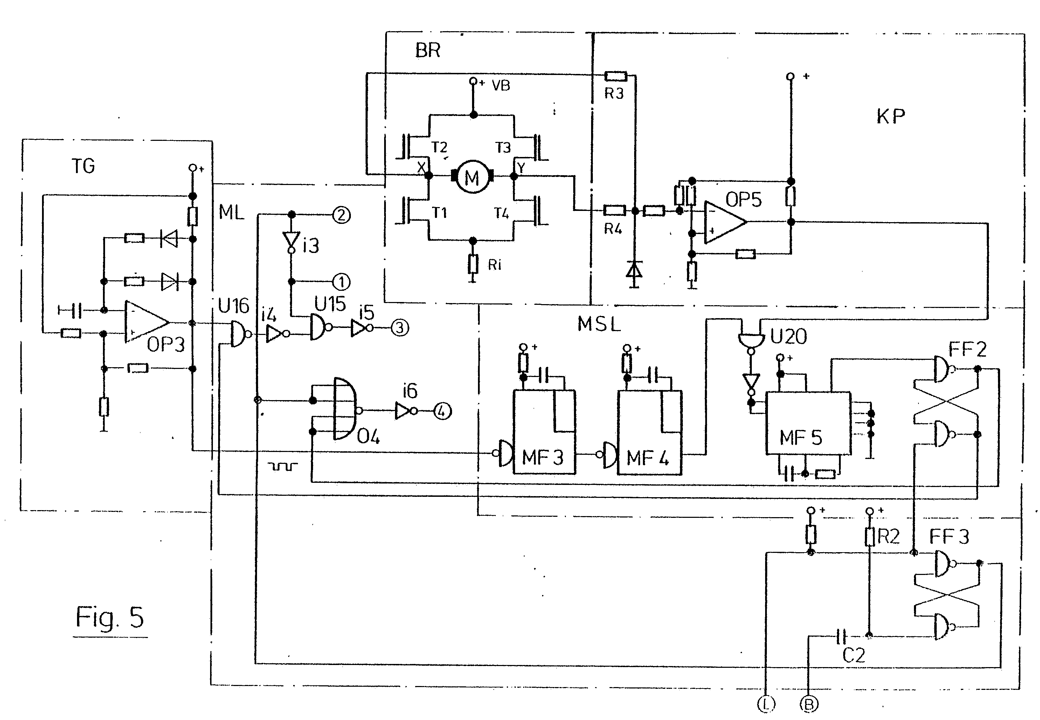

In Fig. 5 erkennt man eine erste das Blockschaltbild der Fig. 4 realisierende Schaltungsanordnung. Der Taktgenerator TG besteht aus einem als astabiler Multivibrator geschalteten Operationsverstärker OP3. Durch die Verwendung zweier antiparalleler Dioden im Rückkopplungszweig kann das gewünschte, stark asymmetrische Ausgangsimpulssignal erzeugt werden.

In der Brückenschaltung BR sind vier Feldeffekt-Transitoren T1, T2, T3, T4 angeordnet, in deren einer Diagonale der Motor M liegt. Den anderen Brückendiagonalen wird die Motorbetriebsspannung VB zugeführt, wobei die Verbindung zwischen der Transistorenbrücke und der Bezugselektrode über einen Widerstand Ri erfolgt. An diesem Widerstand Ri kann eine dem Motorstrom proportionale Spannung abgegriffen werden. Diese Spannung kann, wie noch später zu beschreiben sein wird, als Ist-Wert der Drehzahlregelung dienen.

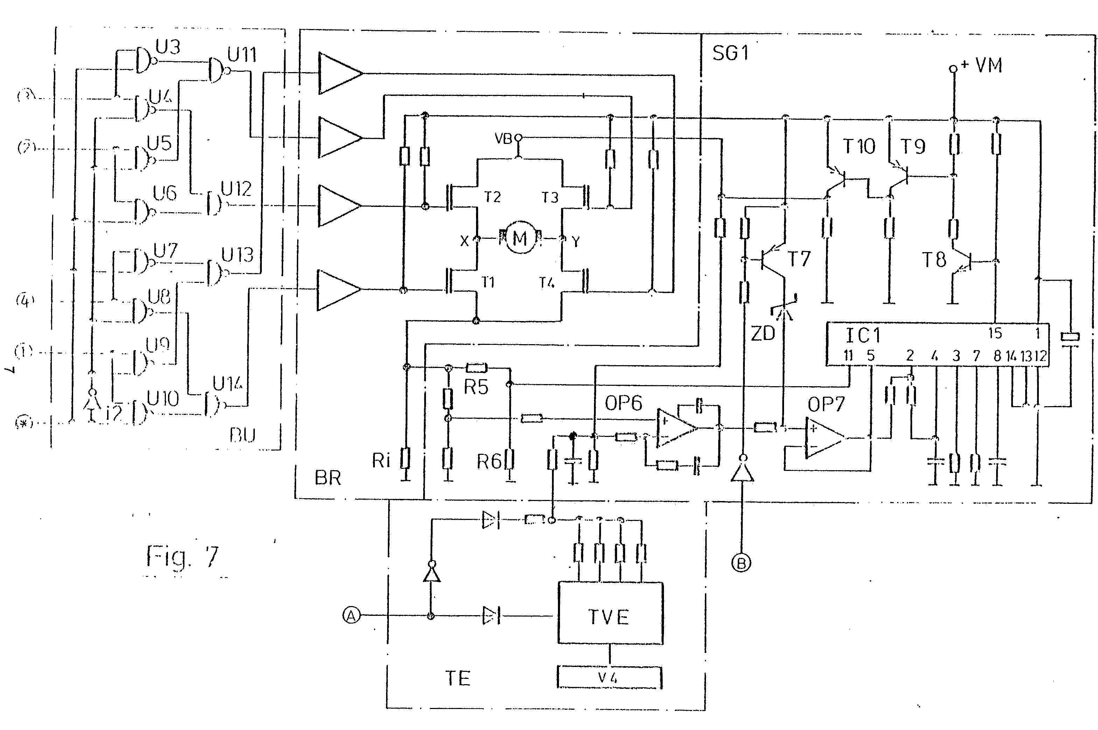

Der der Brückenschaltung BR vorgeschaltete Betriebsartenumschalter BU ist wegen der besseren Übersichtlichkeit nicht in der Fig. 5, sondern erst in der Fig. 7 dargestellt.

Mit den Motoranschlüssen ist über einen Spannungsteiler R3, R4 der Meßeingang eines Nullspannungskomparators KP verbunden. Dabei handelt es sich um einen in bekannter Weise geschalteten Operationsverstärker OP5. Das Ausgangssignal des Nullspannungskomparators KP ist auf ein Nicht-UND-Gatter O20 geführt. Der zweite Eingang dieses Gatters U20, das Teil der Meß-und Stopp-Logik MSL ist, ist über zwei seriengeschaltete Monoflops MF3, MF4 mit dem Ausgang des Taktgenerators TG verbunden. Die beiden Monoflops MF3, MF4 dienen zur Verzögerung und Formung der Ausgangsimpulse des Taktgenerators TG. So lange der Motor M sich dreht, liegt am Ausgang des Komparators KP ein Signal an und die Ausgangsimpulse des Monoflops MF4 gelangen durch das Nicht-UND-Gatter U20 auf ein retriggerbares Monoflop MF5. Während dieser Zeit liegt am Ausgang des Monoflops MF5 ein Dauersignal an, welches ein nachgeschaltetes Flipflop FF2 so schaltet, daß das Nicht-UND-Gatter U16 für die Ausgangsimpulse des Taktgenerators TG durchlässig wird.

Das Flipflop FF2 wird von dem an der Klemme L anliegenden, von der Startschaltung kommenden Startsignal bei Beginn der Motordrehbewegung so gekippt, daß das Nicht-UND-Gatter U16 für die Ausgangsimpulse des Taktgenerators TG, die nur für den Bremsvorgang benötigt werden, undurchlässig wird. Der obere Ausgang des Flipflop FF2 schaltet dann die Motorsteuerlogik ML so, daß je zwei diagonal angeordnete Transistoren T1, T3 ; T2, T4 in der Brückenschaltung BR ein Dauersignal bekommen, je nachdem, ob der Motor M vorwärts oder rückwärts drehen soll.

Zu Beginn des Bremsvorgangs, d. h. beim Zählerstand 0, wird über die Klemme B ein Bremssignal auf ein Flipflop FF3 geschaltet.

Waren zuerst die Transistoren T1 und T3 leitend, so sind es jetzt die Transistoren T2 und T4. Dadurch liegt an dem Motor eine Gegenspannung an. Außerdem wird das Gatter U16 für die Impulse des Taktgenerators TG durchlässig.

Während der Dauer der kurzen Ausgangsimpulsteile des Taktgenerators TG wird über das Nicht-UND-Gatter U16 jeweils der dem Plus-Anschluß der Motorbetriebsspannung VB zugeordnete Transistor T2 oder T3 kurzzeitig abgeschaltet, so daß in dieser Zeitspanne der Motor ungebremst weiterläuft. Der der Bezugselektrode benachbarte, jeweils eingeschaltete Transistor T1 oder T4 bleibt eingeschaltet, so daß mit Hilfe des Nullspannungskomparators KP die Leerlaufspannung des Motors gemessen werden kann. Die Auswertung dieser Messung muß um eine kurze Zeitspanne gegenüber dem Abschalten des Transistors T2 bzw. T3 verzögert werden, damit nicht die in der Motorinduktivität gebildete Spannungsspitze gemessen wird. Diese Verzögerung wird wie schon erwähnt durch die beiden Monoflops MF3, MF4 bewirkt.

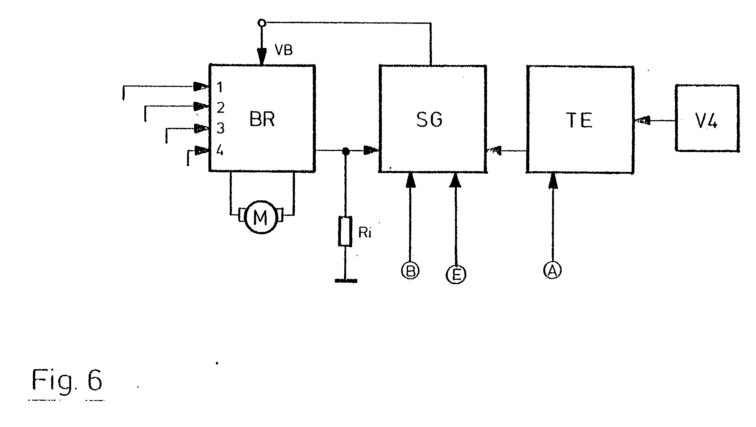

Figur 6 zeigt das Blockschaltbild der Drehzahlregelung. Man erkennt einen vierten Kodierschalter V4, an dem mehrere Geschwindigkeitsstufen vorgewählt werden können. Die Stellung des Kodierschalters V4 beeinflußt einen Tempo-

einsteller TE. Dem Tempoeinsteller TE wird auch das an der Klemme A anstehende Ausgangssignal der Einrichtung zur Erkennung kleiner Volumina KVA zugeführt, mit dem der Tempoeinsteller TE bei kleinen Volumina automatisch auf die kleinste Geschwindigkeitsstufe zurückgestellt wird. Der Tempoeinsteller TE beeinflußt einen Spannungsgenerator SG, der als Regelschaltung ausgebildet ist, und den vom Tempoeinsteller gelieferten Soll-Wert mit dem am Widerstand Ri abfallenden oder über die Klemme E von der optischen Impuls-Integrierschaltung OII gelieferten Ist-Wert vergleicht. Über das an der Klemme B anstehende Bremssignal, welches wie schon erwähnt dann auftritt, wenn der Zähler auf den Zählerstand Null heruntergezählt hat, kann der Spannungsgenerator SG auf einen Bremsspannungswert unabhängig vom Tempoeinsteller TE eingestellt werden. Ausgangsgröße des Spannungsgenerators SG ist die Motorbetriebsspannung VB, die der Brückenschaltung BR zugeführt wird.

Figur 7 zeigt eine erste dem Blockschaltbild der Fig. 6 entsprechende Schaltungsrealisierung, wobei zusätzlich der Betriebsartenumschalter BU dargestellt ist, der hier besser erkennbar wird. Der Betriebsartenumschalter BU besteht aus einem Netzwerk von zwölf Nicht-UND-Gattern, U3 ... U14 und einem Inverter i2. Mit Hilfe des an der Klemme V/R anstehenden Vorwärts-Rückwärts-Signals können entweder die Gatter U3, U6, U7 und U10 oder die Gatter U4, U5, U8 und U9 für die von der Motorsteuerlogik ML kommenden, an den Klemmen 1, 2, 3, 4 anstehenden Steuersignale durchlässig gemacht werden. Die Gatter U11, U12, U13, U14 fassen diese Signale logisch zusammen und führen sie über Pegelumsetzer auf die Steuerelektroden der Brückentransistoren T1, T2, T3, T4.

Der als Drehzahlregler dienende Spannungsgenerator SG1 enthält eingangsseitig einen als PI-Regler geschalteten Operationsverstärker OP6, dessen Plus-Eingang über einen Spannungsteiler mit dem Meßwiderstand Ri der Transistorbrückenschaltung BR und mit dem Ausgang des Tempoeinstellers TE verbunden ist. Der Ausgang des PI-Reglers OP6 ist über einen Entkopplungsverstärker OP7 auf einen Eingang 5 des integrierten Schaltkreises IC1 geführt, an dessen Ausgang 15 eine gepulste Spannung mit einem Puls-Pausen-Verhältnis entsprechend der vorgewählten Geschwindigkeitsstufe und der momentan auftretenden Motorbelastung ansteht. Die gepulste Spannung am Ausgang 15 wird in einem aus den drei Transistoren T8, T9, T10 gebildeten Verstärker verstärkt und als Motorbetriebsspannung VB an die Brückenschaltung BR geliefert. Ein Teil der Motorbetriebsspannung VB wird über einen Spannungsteiler und, geglättet mit Hilfe eines Kondensators, am Minus-Eingang des PI-Reglers OP6 zusammen mit dem Ausgangssignal des Tempoeinstellers gegengekoppelt. Der Innenwiderstand einer derartigen gepulsten Spannungsquelle ist extrem klein im Vergleich zum Motorwiderstand. Außerdem ist der Wirkungsgrad einer derartigen gepulsten Spannungsquelle sehr hoch. Die Regelschaltung selbst beschleunigt den Motor bei Unterdrehzahl und bremst ihn aktiv bei Überdrehzahl. Dies ist beispielsweise immer dann der Fall, wenn von einer hohen auf eine niedrige Geschwindigkeitsstufe umgeschaltet wird.

Bei dem integrierten Schaltkreis IC1 handelt es sich um einen handelsüblichen Steuerschaltkreis für gepulste Netzteile, beispielsweise um den Schaltkreis TDA 1 060, der von der deutschen Firma Valvo verkauft wird. Am Eingang 11 des integrierten Schaltkreises IC1 liegt über einen Spannungsteiler R5, R6 ein Teil der am Meßwiderstand Ri abfallenden Spannung. Diese Spannung am Eingang 11 dient als Maximalstrom-Begrenzung. Sobald die Spannung am Eingang 11 zu hoch zu werden droht, wird das Puls-Pausen-Verhältnis am Ausgang 15 derart verändert, daß die Motorbetriebsspannung VB soweit absinkt, daß der Strom nicht zu groß wird.

Die gewünschte Drehzahlstufe kann über dem Tempoeinsteller TE eingestellt werden. Der Tempoeinsteller TE enthält einen Voreinsteller V4 und einen BCD-Schalter, der mit Hilfe der an seinem Ausgang angeordneten Widerstände den PI-Regler beeinflußt. Mit Hilfe des an der Eingangsklemme A anstehenden Signals für kleine Volumina kann der BCD-Schalter derart umgangen werden, daß immer die kleinste Geschwindigkeitsstufe, d. h. die kleinste Motordrehzahl eingestellt wird.

Sobald an der Klemme B das Bremssignal auftritt, wird ein Transistor T7 eingeschaltet, der über eine in seinem Kollektorkreis liegende Z-Diode ZD die Spannung am Eingang 5 des integrierten Schaltkreises IC1 so beeinflußt, daß er unabhängig von der eingestellten Geschwindigkeitsstufe eine konstante Motorbetriebsspannung VB als Bremsspannung abgibt. Der Motor wird also mit einer hohen Gegenspannung aktiv gebremst. Die Höhe der Bremsspannung wird so eingestellt, daß zusammen mit der in der rotierenden Motorwicklung erzeugten Spannung die zulässige maximale Motorspannung nicht überschritten wird, um die Lebensdauer des Motors nicht zu verringern. Außerdem bleibt die Maximalstrombegrenzung am Eingang 11 des IC1 wirksam.

Figur 8 zeigt ein Ausführungsbeispiel der Kolbenstartpunktsucheinrichtung KSS. An der Klemme C liegen die Ausgangsimpulse der Ortsmeßschaltung OMS an. Über den ersten Eingang des Nicht-ODER-Gatters 021 und einen nachgeschalteten Inverter i1 gelangen sie auf ein retriggerbares Monoflop MF6. Die Zeitkonstante dieses Monoflops MF6 ist so eingestellt, daß der Ausgang so lange Signal führt, wie Impulse vom Ortsmeßsystem OMS erzeugt werden. Bleiben die Impulse des Ortsmeßsystems OMS aus, so verschwindet am Ausang des retriggerbaren Monoflops MF6 nach einer bestimmten Zeitspanne das Signal. Dadurch wird ein Monoflop MF7 gestartet, welches an die Klemme D ein kurzes Signal abgibt. Dieses Signal D wird an die Nullsetzeingänge cl der Zähler Z1, Z2, Z3 geführt, worauf diese auf Null gesetzt werden. Sobald jedoch die Zähler auf Null stehen, wird — wie vorstehend bereits erläutert — der Bremsvorgang eingeleitet und der Motor stillgesetzt.

Zweck der Kolbenstartpunktsucheinrichtung KSS ist es, den Startpunkt des Kolbenhubes dadurch festzulegen, daß der Kolben vom Motor gegen das Zylinderende gedrückt wird. Dies kann beispielsweise beim Einlegen einer neuen Pipette in das Handdosiergerät oder aber beim Übergang von einer Dosierart zu einer anderen nötig werden. Um den Startpunktsuchvorgang auszulösen, wird ein Suchstartsignal PST erzeugt, welches das retriggerbare Monoflop MF6 und — in der Zeichnung nicht dargestellt — den Motor M in Vorwärtsrichtung startet. Sobald der Motor M sich dreht, werden durch den mit dem Kolben gekoppelten Impulsmaßstab IM in der Ortsmeßschaltung OMS Impulse erzeugt, die das retriggerbare Monoflop MF6 so lange in seinem labilen Zustand halten, bis der Kolben am Zylinder anstößt.

Ein weiterer Vorteil der Kolbenstartpunktsucheinrichtung KSS besteht darin, daß auch dann, wenn der Kolben durch einen Defekt an seiner freien Bewegung gehindert wird, der Motor abgeschaltet wird, so daß eine Überlastung nicht eintreten kann.

Fig. 9 zeigt anhand der Kurven a und b den Verlauf der Drehzahl n des Motors M über der Zeit t bei verschieden eingestellten Soll-Drehzahlen und Dosiervolumina. Wie die Kurve a zeigt, wird der Motor zum Zeitpunkt t0 von Null an beschleunigt, bis er etwa zum Zeitpunkt t1 seine Soll-Drehzahl n3 erreicht hat. Mit dieser Drehzahl läuft er weiter, bis zum Zeitpunkt t2 die Erkennungsschaltung für kleine Volumina ein Ausgangssignal an die Klemme A abgibt, wodurch der Drehzahlregler auf die niedrigste Geschwindigkeitsstufe n1 eingestellt wird. Dementsprechend verringert sich die Drehzahl n bis auf den Wert n1. Zum Zeitpunkt t3 ist der voreingestellte Dosierhub erreicht, der Zähler hat den Wert Null erreicht, und der Motor wird schlagartig stillgesetzt. Dementsprechend fällt die Drehzahl auf O ab.

Die Kurve b zeigt den Verlauf der Drehzahl, wenn nur ein sehr kleines Volumen eingestellt ist. Vom Zeitpunkt to an beschleunigt der Motor entsprechend der Kurve, die für die niedrigste Geschwindigkeitsstufe n1 charakteristisch ist. Diese Enddrehzahl erreicht er jedoch nicht, da bereits zum Zeitpunkt t4 das voreingestellte Volumen erreicht ist, der Zähler den Wert Null aufweist und der Motor schlagartig auf die Drehzahl O heruntergebremst wird.

Fig. 10 zeigt ein Ausführungsbeispiel für die optische Impulsintegrierschaltung OII. An der Klemme C laufen die Ausgangs-Impulse der Ortsmeßschaltung OMS auf den als Integrierer geschalteten Operationsverstärker OP8. Die an der Klemme E anstehende Ausgangsspannung ist direkt proportional der Zahl der pro Zeiteinheit an der Klemme C ankommenden Impulse, die wiederum der Geschwindigkeit entspricht, mit der der Kolben und der an ihm befestigte Impulsmaßstab bewegt werden.

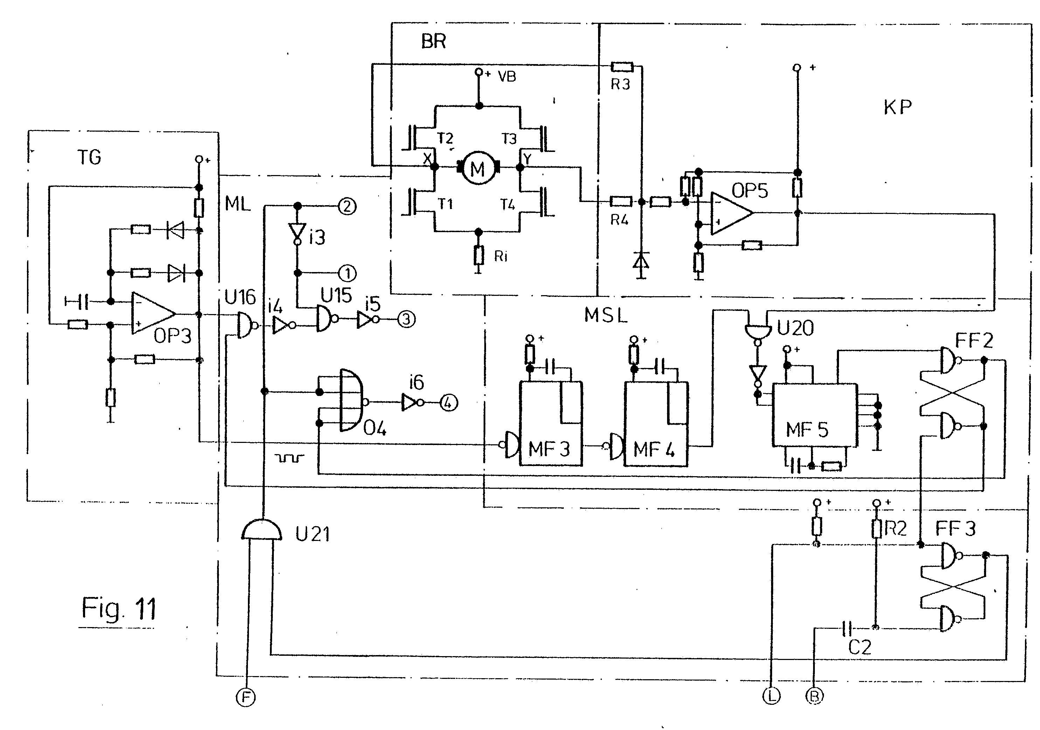

Fig. 11 zeigt eine zweite dem Blockschaltbild der Fig. 4 entsprechende Schaltungsrealisierung, die sich von der der Fig. 5 nur dadurch unterscheidet, daß ein UND-Gatter U21 in die von dem Flipflop FF3 kommende Leitung eingeschaltet ist. Der zweite Eingang des UND-Gatters U21 ist mit einer Klemme F verbunden, an der die aus dem Eingang 15 der integrierten Schaltung IC1 kommende gepulste Spannung, deren Puls-Pausen-Verhältnis der vorgewählten Geschwindigkeitsstufe und der momentan auftretenden Motorbelastung entspricht, ansteht. Mit Hilfe des Gatters U21 gelingt es also, die Transistoren T1, T3 bzw. T2, T4 der Brückenschaltung BR so zu takten, daß sich am Motor M eine Spannung einstellt, die der vorgewählten Geschwindigkeitsstufe und der momentanen Motorbelastung entspricht, ohne daß ein gesonderter Spannungsverstärker, wie er in der Fig. 7 durch die Transistoren T8, T9, T10 gebildet wird, nötig wäre. Die Transistoren T1 ... T4 der Brückenschaltung sowie die ihnen vorgeschalteten Gatter und Pegelwandler sind gleichzeitig Teil der getakteten Spannungsquelle.

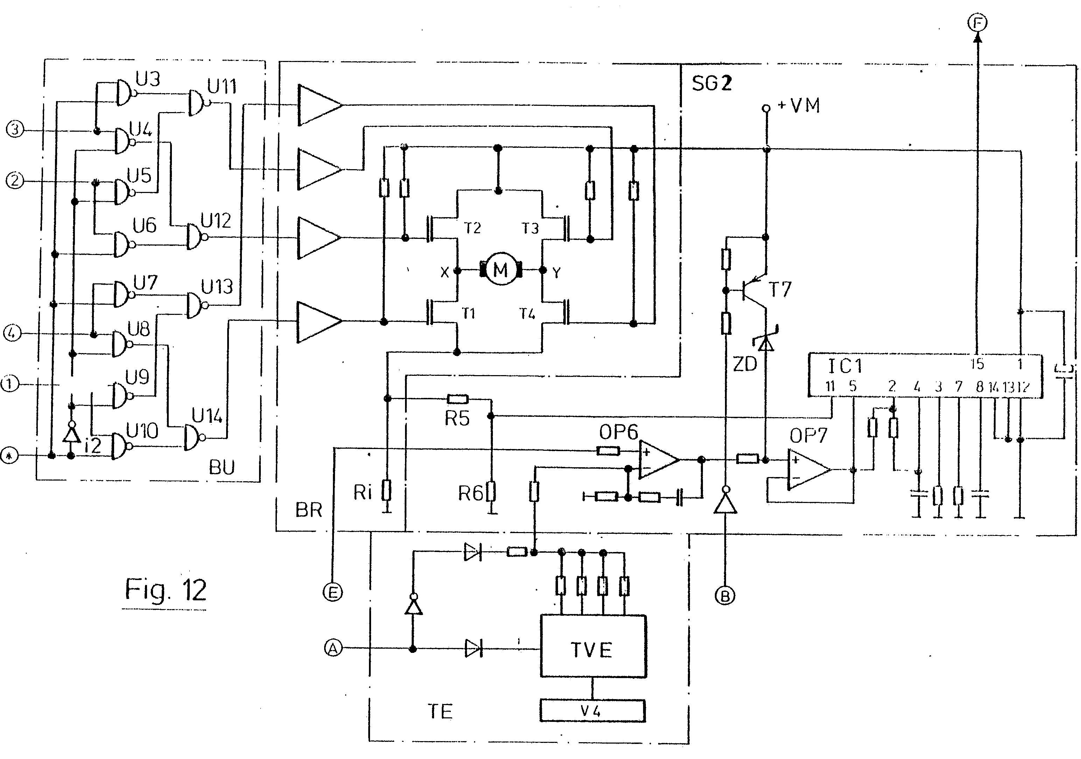

Den Aufbau des abgewandelten Spannungsgenerators SG2 zeigt Fig. 12. Man erkennt den als PI-Regler geschalteten Operationsverstärker OP6, an dessen Plus-Eingang das von der Klemme E kommende Ausgangssignal der optischen Impulsintegrierschaltung OII, welches der momentanen Geschwindigkeit des Kolbens direkt proportional ist, anliegt. Am Minus-Eingang des PI-Reglers OP6 liegt das Ausgangssignal des Tempoeinstellers TE als Soll-Wert an. Die Ausgangsspannung des PI-Reglers OP6 steuert über den als Pufferverstärker geschalteten Operationsverstärker OP7 den integrierten Schaltkreis IC1. Der Ausgang 15 des integrierten Schaltkreises IC1 ist an die Klemme F geführt, die mit dem zweiten Eingang des UND-Gatters U21 verbunden ist. Dem Eingang 11 des integrierten Schaltkreises IC1 ist wiederum über den Spannungsteiler R5, R6 ein Teil der am Meßwiderstand Ri abfallenden Spannung für die Maximalstrombegrenzung zugeführt. Das obere Ende der Transistorbrückenschaltung ist bei dieser Ausführungsform direkt mit der ungeregelten Motorversorgungsspannung VM verbunden.

Anhand eines Zahlenbeispiels soll die mit der erfindungsgemäßen Dosiervorrichtung erreichbare Genauigkeit des Dosiervorgangs erläutert werden. Eine praktisch erprobte erfindungsgemäße Dosiervorrichtung hat eine Hublänge von 60 mm, wobei je nach Dimensionierung der Pipettenspitze Volumina von 0,005 ml bis 12,5 ml dosiert werden können. Die Anhaltgenauigkeit der Vorrichtung ist besser als 0,02 mm. Das sind 0,033 % der Hublänge. Diese Genauigkeit ist so gut, daß die Genauigkeit der Volumendosierung praktisch nur noch von der Präzision der verwendeten Spitzen abhängt. Die Gesamtdauer eines Pipettiervorgangs liegt dabei bei etwa 3-5 Sekunden.

**Ansprüche**

1. Dosiervorrichtung für Flüssigkeiten, insbesondere in der klinischen Chemie, mit einem mit einem Motor (M) angetriebenen Kolben-Zylinder-System, mit einem optischen Ortsmeßsystem (OMS), dessen transparenter, mit undurchsichtigen Markierungen versehener Impulsmaßstab (IM) am Kolben befestigt ist, mit einer optisch-digitalen, einen Lichtsender (PS) und einen Lichtempfänger (PE) zur optischen Abtastung der Impulse einschließenden Ortsmeßschaltung, die den Hub des Kolbens in eine entsprechende Anzahl von digitalen Impulsen umformt, und mit einer Steuer-, Rechen- und Vergleicherschaltung, die eine die digitalen Impulse zählende Zähleinrichtung (VEZ) enthält und den Hub des Kolbens in Abhängigkeit von der vorgewählten Einstellung des Dosiervolumens steuert, dadurch gekennzeichnet, daß benachbart zum ersten Impulsmaßstab (IM) ein weiterer Impulsmaßstab als Abtastplatte (AP) in einer relativ zum Zylinder festen Position angeordnet ist, daß die Markierungen auf den Impulsmaßstäben (IM, AP) ein regelmäßiges Strichgitter bilden, daß der Motor (M) ein Gleichstrommotor ist, daß der Motor (M) in Abhängigkeit vom Stand der Zähleinrichtung (VEZ) in seiner Drehzahl gesteuert ist, derart, daß bei Annäherung an das vorgewählte Dosiervolumen eine niedrigste Geschwindigkeitsstufe eingestellt ist und daß ein Schaltungsteil zum schlagartigen Stillstehen des Motors (M) unter Anlegen einer Gegenspannung bei Erreichen des dem vorgewählten Dosiervolumen entsprechenden Zählerstandes vorgesehen ist.

2. Vorrichtung nach Anspruch 1, dadurch gekennzeichnet, daß im optischen Ortsmeßsystem (OMS) der Lichtempfänger (PE) ein Phototransistor, der Lichtsender (PS) eine Lumineszensdiode ist, daß dem Lichtempfänger (PE) ein Spannungskomparator (OP2) nachgeschaltet ist und daß zwischen Lichtempfänger (PE) und Spannungskomparator (OP2) ein Differenzierglied (R1, C1) eingeschaltet ist.

3. Vorrichtung nach Anspruch 1 oder 2, dadurch gekennzeichnet, daß die Steuer-, Rechen- und Vergleicherschaltung eine Drehzahlregelung enthält, die den Motor (M) auf die vorgewählte Geschwindigkeitsstufe beschleunigt und ihn eine bestimmte Zahl von optischen Impulsschritten vor Erreichen des vorgewählten Volumens auf die niedrigste Geschwindigkeitsstufe verzögert.

4. Vorrichtung nach Anspruch 3, dadurch gekennzeichnet, daß die Drehzahlregelung eine geregelte Spannungsquelle (SG) enthält, deren Ausgangsspannung die Motorbetriebsspannung (VB) ist und deren Sollwert in mehreren Stufen einstellbar ist und daß die Drehzahlregelung bei einem kleinen zu verarbeitenden Volumen immer auf die niedrigste Geschwindigkeitsstufe eingestellt wird.

5. Vorrichtung nach wenigstens einem der Ansprüche 1 bis 4, dadurch gekennzeichnet, daß während des Abbremsens des Motors (M) von der niedrigsten Geschwindigkeitsstufe zum Stillstand der maximal zulässige Wert der Motorsbetriebsspannung (VB) nicht überschritten wird.

6. Vorrichtung nach wenigstens einem der Ansprüche 1 bis 5, dadurch gekennzeichnet, daß während des Stillsetzens des Motors (M) unter Anlegen einer Gegenspannung die Gegenspannung periodisch abgeschaltet und währenddessen die Leerlaufspannung des Motors (M) gemessen wird.

7. Vorrichtung nach Anspruch 6, dadurch gekennzeichnet, daß die Leerlaufspannung des Motors (M) mit einem Nullspannungskomparator (KP) gemessen wird, der bei Verschwinden der Leerlaufspannung ein Ausgangssignal abgibt.

8. Vorrichtung nach Anspruch 7, dadurch gekennzeichnet, daß der Meßeingang des Nullspannungskomparators (KP) über einen Spannungsteiler (R3, R4) mit den Motoranschlüssen (X, Y) verbunden ist.

9. Vorrichtung nach Anspruch 8, dadurch gekennzeichnet, daß der Nullspannungskomparator (KP) ein beschalteter Operationsverstärker (OP5) ist.

10. Vorrichtung nach wenigstens einem der Ansprüche 1 bis 9, dadurch gekennzeichnet, daß der Motor (M) in einer Brückenschaltung (BR) von vier Transistoren (T1, T2, T3, T4) angeordnet ist.

11. Vorrichtung nach Anspruch 10, dadurch gekennzeichnet, daß in der Stromzuführung zur Transistorbrückenschaltung (BR) ein Widerstand (Ri) angeordnet ist, und daß die an diesem Widerstand (Ri) abfallende Spannung als Ist-Wert für die Drehzahlregelung bzw. die Strombegrenzung dient.

12. Vorrichtung nach Anspruch 10 oder 11, dadurch gekennzeichnet, daß der Transistorbrückenschaltung (BR) ein Betriebsartenumschalter (BU) zum Umschalten des Motors (M) auf Vorwärts- oder Rückwärtslauf (V/R) vorgeschaltet ist.

13. Vorrichtung nach Anspruch 12, dadurch gekennzeichnet, daß dem Betriebsartenumschalter (BU) eine Motorsteuerlogik (ML) vorgeschaltet ist, die in der Motorbetriebsphase jeweils zwei diagonal zueinander liegende Transistoren (T1, T3 ; T2, T4) einschaltet, während der Motorstillsetzphase einen der beiden Transistoren (T2 ; T3) periodisch kurzzeitig abschaltet und bei Motorstillstand den Motor kurzschließt.

14. Vorrichtung nach wenigstens einem der Anspruch 10 bis 13, dadurch gekennzeichnet, daß zum periodischen, kurzzeitigen Abschalten eines der beiden den Bremsstrom führenden Transistoren (T2 ; T3) ein Taktgenerator (TG) vorgesehen ist, der stark asymmetrische Impulse abgibt, wobei die langen Impulse die Bremsphase, die kurzen Impulse die Meßphase definieren.

15. Vorrichtung nach Anspruch 14, dadurch gekennzeichnet, daß während der Dauer der kurzen Impulse in der Motorstillsetzphase der eine der beiden Bremsstrom führenden Transistoren (T2 ; T3) abgeschaltet, während der Dauer der langen Impulse eingeschaltet ist.

16. Vorrichtung nach wenigstens einem der Ansprüche 14 oder 15, dadurch gekennzeichnet, daß eine Meß und Stop-Logik (MSL) vorgesehen ist, die während der Dauer der kurzen Impulse des Taktgenerators (TG) den Meßzeitpunkt festlegt und während der Motorbremsphase solange Impulse an die Motorsteuerlogik (ML) weiterleitet, bis das Nullspannungssignal des Nullspannungskomparators (KP) erscheint.

17. Vorrichtung nach wenigstens einem der Ansprüche 1 bis 16, dadurch gekennzeichnet, daß die Zähleinrichtung (VEZ) aus einem extern voreinstellbaren rückwärts zählenden Zähler (Z), einer extern bedienbaren Hand-Starteinrichtung (ST) für den Rückwärtszähler (Z), einem Zähleingang (ZE) für die Impulse von dem Ortsmeßsystem (OMS) und einer Einrichtung (KVA) zum Erkennen kleiner Volumina besteht, deren Ausgang (A) mit der Drehzahlregelschaltung verbunden ist und diese auf die kleinste Geschwindigkeitsstufe einstellt.

18. Vorrichtung nach wenigstens einem der Ansprüche 1 bis 17, dadurch gekennzeichnet, daß eine vollautomatisch wirkende Kolbenstartpunktsucheinrichtung (KSS) vorgesehen ist, die die Zahl der pro Zeiteinheit erzeugten optischen Impulsschritte überwacht und den Motor (M) abschaltet, wenn pro Zeiteinheit keine Impulse erzeugt werden.

19. Vorrichtung nach wenigstens einem der Ansprüche 1-18, dadurch gekennzeichnet, daß die Zahl der Ausgangsimpulse des Ortsmeßsystems (OMS) pro Zeiteinheit als Maß der Kolbengeschwindigkeit zur Regelung der Drehzahl des Motors (M) verwendet wird.

20. Vorrichtung nach Anspruch 19, dadurch gekennzeichnet, daß ein Optischer-Impuls-Integrator (OII) vorgesehen ist, der die Ausgangsimpulse des Ortsmeßsystems (OMS) integriert und als der Kolbengeschwindigkeit proportionale Spannung an die geregelte Spannungsquelle (SG2) für die Motorbetriebsspannung (VB) weiterleitet.

21. Vorrichtung nach wenigstens einem der Ansprüche 4 bis 20 dadurch gekennzeichnet, daß die geregelte Spannungsquelle (SG1, SG2) für die Motorbetriebsspannung (VB) als getaktete Spannungsquelle mit einstellbarem Puls-Pausen-Verhältnis ausgebildet ist.

22. Vorrichtung nach Anspruch 21, dadurch gekennzeichnet, daß die Transistoren (T1, T2, T3, T4) der Brückenschaltung (BR) gleichzeitig Teil der getakteten Spannungsquelle (SG2) sind.

## Claims

1. Measuring device for liquids, especially in clinical chemistry, with a piston-cylinder system operated with a motor (M), with an optical positional measurement system (OMS), the transparent impulse scale (IM) of which is provided with non-transparent markings and fixed to the piston, with an optical-digital positional measurement circuit including a light emitter (PS) and a light receiver (PE) for the optical sensing of the impulses, which positional measurement circuit converts the stroke of the piston into a corresponding number of digital impulses, and with a control, calculation and comparison circuit, which contains a counter device (VEZ) counting the digital impulses and controls the stroke of the piston in dependence upon the preselected adjustment of the dosing volume, characterised in that adjacent the first impulse scale (IM) there is arranged a further impulse scale as sensing plate (AP) in a position fixed relative to the cylinder, that the markings on the impulse scales (IM, AP) form a regular lined grating, that the motor (M) is a direct current motor, that the motor (M) is, in its speed of rotation, controlled in dependence upon the status of the counter device (VEZ) in such a manner that, in the case of proximation to the preselected measuring volume, a lowermost speed phase is adjusted and that a circuit component is provided for the sudden stopping of the motor (M) with application of a countercurrent upon reaching the counter state corresponding to the preselected measuring volume.

2. Device according to claim 1, characterised in that, in the optical positional measurement system (OMS), the light receiver (PE) is a photo transistor, the light emitter (PS) a luminescent diode, that after the light receiver (PE) there is inserted a voltage comparator (OP2) and that between light receiver (PE) and voltage comparator (OP2) there is inserted a differentiation member (RI, CI).

3. Device according to claim 1 or 2, characterised in that the control, calculation and comparison circuit contains a speed regulating means which accelerates the motor (M) to the preselected speed phase and reduces it to the lowermost speed phase a definite number of optical impulse steps before reaching the preselected volume.

4. Device according to claim 3, characterised in that the speed regulating means contains a regulated voltage source (SG), the output voltage of which is the motor operational voltage (VB) and the desired value of which is regulatable in several phases and that the rotational speed regulation is, in the case of processing a small volume, always adjusted to the lowermost speed phase.

5. Device according to at least one of claims 1 to 4, characterised in that during the braking of the motor (M) from the lowermost speed phase to stopping, the maximum admissible value of the motor operational voltage (VB) is not exceeded.

6. Device according to at least one of claims 1 to 5, characterised in that during the stopping of the motor (M) with application of a countervoltage, the countervoltage is periodically switched off and during this the idling voltage of the motor (M) is measured.

7. Device according to claim 6, characterised in that the idling voltage of the motor (M) is measured with a zero voltage comparator (KP) which, in the case of disappearance of the idling voltage, emits an output signal.

8. Device according to claim 7, characterised in that the measurement input of the zero voltage comparator (KP) is connected with the motor connections (X, Y) via a potentiometer (R3, R4).

9. Device according to claim 8, characterised in that the zero voltage comparator (KP) is a wired-up operation amplifier (OP5).

10. Device according to at least one of claims 1 to 9, characterised in that the motor (M) is arranged in a bridge circuit (BR) of four transistors (T1, T2, T3, T4).

11. Device according to claim 10, characterised in that in the current input to the transistor bridge circuit (BR) there is arranged a resistance (Ri) and that the voltage dropping on this resistance (Ri) serves as the actual value for the speed of rotation regulation or the current limitation.

12. Device according to claim 10 or 11, characterised in that before the transistor bridge circuit (BR) there is inserted a reverser (BU) for the nature of the operation for the switching over of the motor (M) to forwards or backwards running (V/R).

13. Device according to claim 12, characterised in that before the reverser (BU) for the nature of the operation there is inserted a motor control logic (ML) which, in the motor operational phase, in each case switches in two transistors (T1, T3 ; T2, T4) lying diagonally to one another, whereas the motor stopping phase periodically switches off for a short time one of the two transistors (T2 ; T3) and, in the case of motor stoppage, short circuits the motor.

14. Device according to at least one of claims 10 to 13, characterised in that for the periodic, short-period switching off of one of the two transistors (T2 ; T3) conducting the braking current there is provided a rhythmic generator (TG) which gives off strongly asymmetrical impulses, whereby the long impulses define the braking phase, the short impulses the measurement phase.

15. Device according to claim 14, characterised in that during the period of the short impulses in the motor stopping phase one of the two transistors (T2 ; T3) conducting braking current is switched off, during the period of the long impulses is switched on.

16. Device according to at least one of claims 14 or 15, characterised in that a measurement and stop logic (MSL) is provided which, during the period of the short impulses of the rhythmic generator (TG), fixes the measurement point of time and during the motor brake phase further conducts impulses to the motor control logic (ML) until the zero voltage signal of the zero voltage comparator (KP) appears.

17. Device according to at least one of claims 1 to 16, characterised in that the counter device (VEZ) consists of an externally preadjustable, backwardly counting counter (Z), an externally operable manual starting means (ST) for the backcounter (Z), a counter input (ZE) for the impulses from the positional measurement system (OMS) and a device (KVA) for the recognition of small volumes, the outlet (A) of which is connected with the speed of rotation regulating circuit and adjusts this to the smallest speed phase.

18. Device according to at least one of claims 1 to 17, characterised in that a fully automatically working piston starting point seeking device (KSS) is provided which supervises the number of optical impulse steps produced per unit time and switches off the motor when no impulses are produced per unit time.

19. Device according to at least one of claims 1 to 18, characterised in that the number of the output impulses of the positional measurement system (OMS) per unit time is used as measure of the piston rate for the regulation of the speed of rotation of the motor (M).

20. Device according to claim 19, characterised in that an optical impulse integrator (OII) is provided which integrates the output impulses of the positional measurement system (OMS) and, as voltage proportional to the piston rate, passes it on to the regulated voltage source (SG2) for the motor operational voltage (VB).

21. Device according to at least one of claims 4 to 20, characterised in that the regulated voltage source (SG1, SG2) for the motor operational voltage (VB) is formed as a rhythmic voltage source with adjustable pulse-pause ratio.

22. Device according to claim 21, characterised in that the transistors (T1, T2, T3, T4) of the bridge circuit (BR) are simultaneously part of the rhythmic voltage source (SG2).

**Revendications**

1. Dispositif de dosage de liquides, destiné en particulier à la chimie clinique, comprenant : un système piston-cylindre entraîné par un moteur (M), un dispositif de repérage optique (OMS), dont l'échelle graduée pour impulsions (IM), transparente et pourvue de marques opaques, est fixée au piston, un circuit de repérage digital optique comportant un émetteur de lumière (PS) et un récepteur de lumière (PE) pour le balayage optique des impulsions, transformant la course du piston en une quantité correspondante d'impulsions digitales, ainsi qu'un circuit de commande, de calcul et de comparaison, comportant un dispositif de comptage (VEZ) comptant les impulsions digitales et qui commande la course du piston en fonction du réglage présélectionné du volume à doser, caractérisé en ce qu'à proximité de la première échelle graduée pour impulsions (IM) est disposée une autre échelle graduée pour impulsions sous la forme d'une plaque de balayage (AP) en position fixe par rapport au cylindre, en ce que les marques sur les échelles graduées pour impulsions (IM, AP) forment un réseau de traits réguliers, en ce que le moteur (M) est un moteur à courant continu, en ce que le régime du moteur (M) est commandé en fonction de la position du dispositif de comptage (VEZ), de telle façon qu'à l'approche du volume à doser présélectionné l'échelon de vitesse le plus bas soit enclenché, et en ce qu'il est prévu un circuit permettant l'arrêt instantané du moteur (M) par l'application d'une contre-tension au moment où la position du compteur correspondant au volume à doser présélectionné est atteinte.

2. Dispositif selon la revendication 1, caractérisé en ce que dans le dispositif de repérage optique (OMS) le récepteur de lumière (PE) est un phototransistor et l'émetteur de lumière (PS) une diode luminescente, en ce que le récepteur de lumière (PE) est suivi d'un comparateur de tensions (OP2) et en ce qu'un élément différenciateur (R1, C1) est inséré entre le récepteur de lumière (PE) et le comparateur de tensions (OP2).

3. Dispositif selon la revendication 1 ou 2, caractérisé en ce que le circuit de commande, de calcul et de comparaison comporte un régulateur de régime qui accélère le moteur (M) jusqu'à l'échelon de vitesse présélectionné et qui le ralentit jusqu'à l'échelon de vitesse le plus bas, un nombre déterminé d'impulsions optiques avant que le volume présélectionné soit atteint.

4. Dispositif selon la revendication 3, caractérisé en ce que le régulateur de régime comporte une source de tension régulée (SG), dont la tension de sortie forme la tension de fonctionnement du moteur (VB), et dont la valeur nominale est réglable par plusieurs échelons et en ce que le régulateur de régime est toujours réglé à l'échelon de vitesse le plus bas lorsque le volume à traiter est faible.

5. Dispositif selon au moins une des revendications 1 à 4, caractérisé en ce que pendant le freinage du moteur (M), à partir de l'échelon de vitesse le plus bas jusqu'à l'arrêt, la valeur maximale admissible de la tension de fonctionnement (VB) du moteur n'est pas dépassée.

6. Dispositif selon au moins une des revendications 1 à 5, caractérisé en ce que pendant la mise à l'arrêt du moteur (M) par l'application d'une contre-tension, cette contre-tension est périodiquement interrompue et pendant cette interruption la tension de marche à vide du moteur (M) est mesurée.

7. Dispositif selon la revendication 6, caractérisé en ce que la marche à vide du moteur (M) est mesurée au moyen d'un comparateur à tension zéro (KP) donnant un signal de sortie au moment de la disparition de la tension de marche à vide.

8. Dispositif selon la revendication 7, caractérisé en ce que l'entrée de mesure du comparateur à tension zéro (KP) est reliée par l'intermédiaire d'un diviseur de tension (R3, R4) aux bornes (X, Y) du moteur.

9. Dispositif selon la revendication 8, caractérisé en ce que le comparateur à tension zéro (KP) est un montage d'un amplificateur opérationnel.

10. Dispositif selon au moins une des revendications 1 à 9, caractérisé en ce que le moteur (M) est monté dans un pont (BR) de quatre transistors (T1, T2, T3, T4).

11. Dispositif selon la revendication 10, caractérisé en ce que dans l'alimentation en courant du montage en pont de transistors (BR) est disposée une résistance (Ri) et en ce que la tension aux bornes de cette résistance (Ri) sert de valeur effective pour le réglage du régime et pour la limitation du courant.

12. Dispositif selon la revendication 10 ou 11, caractérisé en ce que le montage en pont de transistors (BR) est précédé d'un inverseur de mode de fonctionnement (BU) pour faire tourner le moteur (M) en marche avant ou en marche arrière (V/R).

13. Dispositif selon la revendication 12, caractérisé en ce que l'inverseur de mode de fonctionnement (BU) est précédé d'un circuit logique (ML) pour la commande du moteur qui met pendant la phase de fonctionnement du moteur chaque fois deux transistors diagonalement opposés (T1, T3 ; T2, T4) en circuit, met pendant la phase de la mise à l'arrêt du moteur périodiquement brièvement l'un des deux transistors (T2 ; T3) hors circuit, et met le moteur en court-circuit lorsque celui-ci est arrêté.

14. Dispositif selon au moins une des revendications 10 à 13, caractérisé en ce que pour la mise hors circuit périodique pendant un temps court de l'un des deux transistors (T2 ; T3) parcourus par le courant de freinage, un générateur d'impulsions (TG) est prévu, fournissant des impulsions fortement asymétriques, les impulsions longues définissant la phase de freinage, et les impulsions courtes définissant la phase de mesure.

15. Dispositif selon la revendication 14, caractérisé en ce qu'au cours de la phase de mise à l'arrêt du moteur, l'un des deux transistors (T2 ; T3) parcourus par le courant de freinage est hors circuit pendant la durée des impulsions courtes, et en circuit pendant la durée des impulsions longues.

16. Dispositif selon au moins une des revendications 14 ou 15, caractérisé en ce qu'il est prévu un circuit logique de mesure et d'arrêt (MSL), qui détermine pendant la durée des impulsions courtes du générateur d'impulsions (TG) l'instant de la mesure et transmet pendant la phase de freinage du moteur des impulsions au circuit logique de commande du moteur (ML) jusqu'à l'apparition du signal de tension zéro du comparateur à tension zéro (KP).

17. Dispositif selon au moins une des revendications 1 à 16, caractérisé en ce que le dispositif de comptage (VEZ) est constitué par un compteur à rebours (Z) avec présélection externe, un dispositif de démarrage (ST) manuel avec commande externe pour le compteur à rebours (Z), une entrée de comptage (ZE) pour les impulsions du dispositif de repérage (OMS) et par un dispositif (KVA) pour la reconnaissance de faibles volumes, dont la sortie (A) est reliée au circuit de réglage du régime, qu'il règle à l'échelon de vitesse le plus bas.

18. Dispositif selon au moins une des revendications 1 à 17, caractérisé en ce qu'il est prévu un dispositif chercheur du point de démarrage du piston (KSS) entièrement automatique, qui surveille le nombre d'impulsions optiques créées par unité de temps et qui met le moteur (M) hors circuit lorsqu'aucune impulsion n'est créée par unité de temps.

19. Dispositif selon au moins une des revendications 1 à 18, caractérisé en ce que le nombre d'impulsions à la sortie du dispositif de repérage (OMS) par unité de temps est employé comme mesure pour la vitesse du piston pour le réglage du régime du moteur (M).

20. Dispositif selon la revendication 19, caractérisé en ce qu'il est prévu un intégrateur d'impulsions optiques (OII) qui intègre les impulsions de sortie du dispositif de repérage (OMS) et transmet le résultat sous la forme d'une tension proportionnelle à la vitesse du piston, à la source de tension régulée (SG2) pour la tension de fonctionnement (VB) du moteur.

21. Dispositif selon au moins une des revendications 4 à 20, caractérisé en ce que la source de tension régulée (SG1, SG2) pour la tension de fonctionnement du moteur est réalisée sous la forme d'une source de tension à impulsions dont le rapport impulsion/pause est ajustable.

22. Dispositif selon la revendication 21, caractérisé en ce que les transistors (T1, T2, T3, T4) du circuit en pont (BR) font en même temps partie de la source de tension (SG2) à impulsions.

Fig. 1

Pipette

Motorsteuer-logik

Brems - und Drehzahlregelung

Stromversorgung Motor

Getriebe

Glockenanker-motor

Nullspannungs-Komparator

Akkumulator

Impulsmaßstab
Abtastplatte

Impuls-überwachung

optisches Ortsmeß-system

Stromversorgung Elektronik

Netzgerät

Impulsformer

Zähler

Kodierschalter
Volumen

1 2 3

Kodierschalter
Drehzahl

4

0 025 575

Fig. 2

Fig. 3

Fig. 4

Fig. 5

0 025 575

VB

1
2  BR
3
4

M

Ri

SG

TE

V4

Ⓑ  Ⓔ

Ⓐ

Fig. 6

Fig. 7

Fig. 8

Fig. 9

Fig.10

Fig. 11

Fig. 12